# EUROPEAN PATENT APPLICATION

(11) **EP 2 476 416 A1**
(43) Date of publication of application: **18.07.2012**
(21) Application number: 12156951.1
(22) Date of filing: 27.09.2007
(51) Int. Cl.: A61K 31/192, A61P 29/00

(54) **Method of Treatment for Inflammatory Bowel Disease**

(30) Priority: 17.11.2006 US 866401 P
(62) Divisional of application: 07843369.5
(71) Applicant: Shire Development Inc., Wayne, PA 19087 (US)
(72) Inventor: Karistadt, Meyeroff Robyn Gail, Florence, KY Kentucky KY 41042 (US); Diebold, Ronald Joseph, Florence, KY Kentucky KY 41042 (US); Pierce, David Montague, Basingstoke, RG24 8EP (GB); Martin, Patrick T, Florence, KY Kentucky KY 41042 (US)
(74) Representative: Harrison Goddard Foote

(57) **Abstract**

The present invention provides methods of treating inflammatory bowel disease comprising administering to a subject in which remission has not been achieved after a first treatment of about 4-8 weeks of 5-ASA (mesalamine or 5-ASA), a further daily dose greater than about 4 g of 5-ASA in controlled release form to achieve a favorable response to treatment.

## Description

This application claims priority to U.S. Provisional Application No. 60/866,401, filed November 17, 2006, the entire contents of which are incorporated herein by reference.

### Background

Inflammatory bowel disease (IBD) is a term used in the art to generically encompass diseases of the intestines such as ulcerative colitis (UC), irritable bowel syndrome, irritable colon syndrome and Crohn's disease (CD). For many of these diseases, in particular CD, the etiology of the disease (bacterial, viral, genetic, or autoimmune) is unknown. Inflammatory bowel diseases such as CD or UC have been treated in the past with salicylic acid derivatives (such as 5-aminosalicylic acid, also known as 5-ASA, mesalamine or mesalazine; salts or esters of 5-ASA; and prodrugs of 5-ASA, such as sulfasalazine). 5-ASA is used as a first-line treatment for mild-to-moderate ulcerative colitis.

It is known that doses of 5-ASA >2g/day are no more effective than lower doses for maintaining remission in patients with UC (Travis S, Nature Clinical Practice Gastroenterology & Hepatology (2005) 2,564-565). Orally administered 5-ASA acts locally from the luminal side of the inflamed bowel after absorption by the colonic and ileal mucosa, and is primarily acetylated to its major metabolite N-acetyl-5-ASA (Ac-5-ASA) in the gut wall and the liver. Current guidelines for the treatment of active mild-to-moderate UC suggest that oral 5-ASA treatment, as either monotherapy or in combination with a topical formulation, should be prescribed for approximately 4 to 6 weeks for the induction of remission (Carter MJ, et al. Gut 2004;53:V1-16. and Kornbluth A, et al. Am J Gastroenterol 2004; 99:1371-85).

However, if patients fail to achieve remission in this induction phase, there is no suggestion that continued 5-ASA therapy under these conditions will result in the induction of remission. Therefore, the clinician must decide the next appropriate step in the treatment paradigm, which is generally known as "step-up" therapy or "therapy escalation." The next "step" in this paradigm is generally corticosteroid therapy, a therapy often not tolerated by patients due to its side effects.

Patients achieving remission from active, mild-to-moderate UC are generally maintained in remission using continued 5-ASA (aminosalicylate) therapy (Kornbluth A, et al. Am J Gastroenterol 2004; 99:1371-85).

### Summary of the Invention

The present invention involves a method of treatment of a subject suffering from inflammatory bowel disease such as ulcerative colitis (UC), comprising administering an initial daily dose of about 2.4 - 4.8 g of 5-ASA in controlled release form and, when remission is not achieved after such treatment for about 4-8 weeks, changing the initial dose to a daily dosage equal to or greater than about 4g of 5-ASA in controlled release form for a period sufficient to achieve response to treatment.

Once embodiment of the present invention provides a treatment for the induction of remission in a patient suffering from inflammatory bowel disease who has been unresponsive to 5-ASA administered at an oral daily dose of 2.4 g in controlled release form, administered as an oral 1.2 g twice-daily dose, a 2.4 g once-daily dose or a 0.8 g thrice-daily dose, wherein the daily dose is changed to a 4.8 g once-daily dose for a period of up to 8 weeks until the patient treated is in remission.

An alternate embodiment of the present invention provides a method of treating a patient suffering from ulcerative colitis or other inflammatory bowel disease who has been unresponsive to 5-ASA administered at an oral once-daily dose of 4.8 g, comprising administering to the patient a daily dose of 4.8 g of 5-ASA in controlled release form for a period of up to 8 weeks, until the patient treated is in remission.

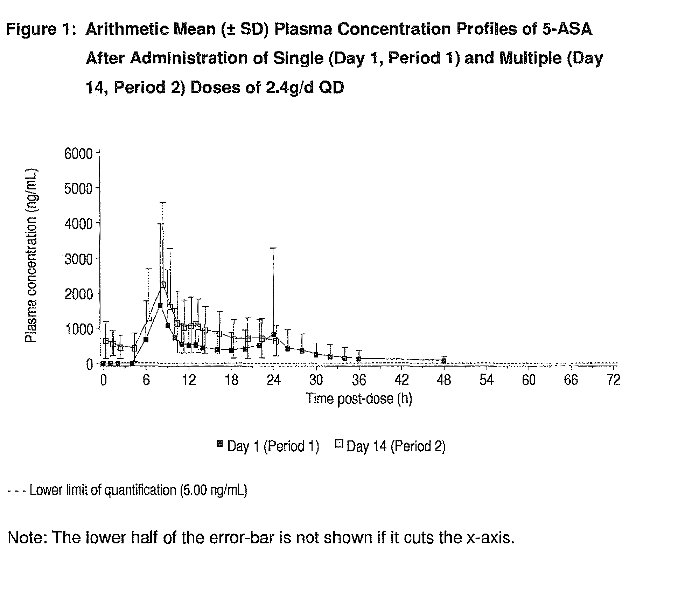

Formulations useful in the method of treatment of the present invention exhibit a single dose *in vivo* plasma concentration profile substantially the same as that shown in Figure 1 and deliver the 5-ASA in a controlled-release manner.

### Brief Description of the Drawings

**Figure 1** depicts the arithmetic mean (± SD) plasma concentration profiles of 5-ASA after administration of single (day 1, period 1) and multiple (day 14, period 2) doses of 2.4g/d QD of MMX® mesalamine) in normal health subjects. Solid, filled-in squares are the data from day 1 (period 1); open, unfilled squares are the data from day 14 (period 2). The dotted line represents the lower limit of quantification (5.00 ng/mL). The X-axis is time post-dose in hours and the Y axis is plasma concentration in ng/mL.

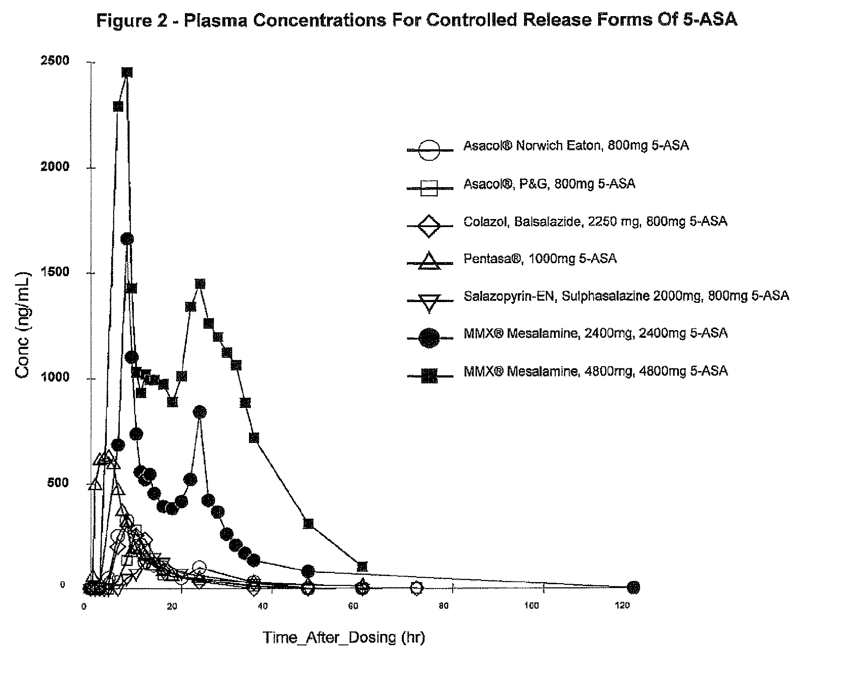

**Figure 2** illustrates the plasma concentrations for controlled release formulations of 5-ASA. Open, unfilled circles represent Asacol® (Norwich Eaton and Procter & Gamble), 800 mg 5-ASA; open, unfilled squares represent Asacol® (Norwich Eaton and Procter & Gamble), 800 mg 5-ASA; open, unfilled rectangles represent Colazol, balsalazide 2250 mg (Salix) (800 mg 5-ASA); open, unfilled triangles represent Pentasa®, 1000 mg (Shire Pharmaceuticals) 5-ASA; open, unfilled, inverted triangles represent Salazopyrin-EN, sulphasalazine 2000 mg (Watson Laboratories) (800 mg 5-ASA); solid, filled circles represent MMX® 5-ASA (Lialda®, 2400 mg 5-ASA) (Cosmo S.p.A. of Milan); and solid, filled squares represent MMX® (Lialda®, 4800 mg 5-ASA) (Cosmo S.p.A. of Milan). The X-axis is time after dosing in hours and the Y axis is plasma concentration of 5-ASA in ng/mL. All concentration levels shown are after only a single dose of medication was administered (*i.e*., after day 1 administration).

### Detailed Description of the Invention

### Definitions

As used herein, the following terms have the meanings given below:
Subjects or patients refer to mammals suffering from inflammatory bowel disease, including but not limited to humans.

Inflammatory bowel disease refers to diseases of the intestines including ulcerative colitis (UC) (including mild-to-moderate ulcerative colitis which has a score of 4-10 on the UC-disease activity index (UC-DAI), with a sigmoidoscopy score of ≥ 4 and a Physician's Global Assessment (PGA) score of ≥ 2), irritable colon syndrome and Crohn's disease.

Controlled release forms of 5-ASA include any orally administrable dosage form which exhibits a single dose *in vivo* plasma concentration profile substantially the same as that shown in Figure 1 and delivers the 5-ASA in a controlled-release manner. Various controlled release dosage forms of 5-ASA are known in the art, including Asacol® (Norwich Eaton and Procter & Gamble), Colazol (Salix), Balsalazide (Salix), Pentasa® (Shire Pharmaceuticals), Salazopysin-EN sulphasalazine (Watson Laboratories) and MMX® Lialda® (Cosmo S.p.A. of Milan)

An exemplary controlled release form of mesalamine) is MMX Multi Matrix System^{®}(MMX®) mesalamine) (Lialda™, also known as Mezavant™ XL in the UK and Ireland, and Mezavant™ elsewhere), described in US patent 6,773,720 to Villa et al., which is incorporated herein by reference. While no specific dosage is required for MMX® mesalamine, an exemplary MMX® mesalamine is a controlled-release oral pharmaceutical composition containing as an active ingredient 1.2 grams of 5-ASA, comprising a multimatrix core consisting of a lipophilic matrix and a hydrophilic matrix wherein the active ingredient is dispersed. In the exemplary formulation, the active ingredient is present in an amount over 80%, generally 80-95%, by weight of the total composition.

The exemplary controlled-release formulation comprises a) an inner lipophilic matrix of unsaturated and/or hydrogenated fatty acid, salts, esters or amides thereof, fatty acid mono-, di- or triglycerides, waxes, ceramides, and cholesterol derivatives with melting points below 90 °C., wherein the active ingredient is dispersed in both the lipophilic matrix and the hydrophilic matrix; b) an outer hydrophilic matrix wherein the lipophilic matrix is dispersed, and wherein the outer hydrophilic matrix is a polymer of acrylic or methacrylic acid, an alkylvinyl polymer, a hydroxyalkyl cellulose, carboxyalkyl cellulose, polysaccharide, dextrin, pectin, starch or starch derivative, alginic acid, or a natural or synthetic gum; and c) optionally other excipients.

Approved treatments of inflammatory bowel disease with 5-ASA utilize daily doses of 2.4 or 4.8 g of the controlled release dosage forms. As indicated above, when such treatments do not achieve remission, in accordance with the present invention a controlled release form of 5-ASA is administered in a daily dosage equal to or greater than about 4 g, preferably about 4-5 g, generally for about 4 to 8 weeks, to achieve remission. In accordance with the preferred embodiments hereof, the treatment of the invention is employed with the currently approved controlled release regimens for the treatment of inflammatory bowel disease, namely, a 2.4 g daily dose (administered in equal amounts once, twice or three times a day) or a 4.8 g daily dose (administered once a day) when remission is not achieved after about 4-8 weeks of such treatments. Utilizing the approved controlled release dosage forms, the treatment of the present invention is usually carried out with a 4.8 g daily dose, preferably with the once-daily administration of the requisite controlled release dosage form, to achieve remission.

As further used herein, remission refers to application of the customary markers utilized in clinical practice to assess IBD. For example, a patient is considered to be in remission for UC if a UC-DAI score of ≤1 is obtained, with rectal bleeding and stool frequency scores of 0, and at least a 1-point reduction in sigmoidoscopy score from baseline. Remission also involves a Physician's Global Assessment (PGA) of ≤1 and no mucosal friability. In some cases, a clinical response in which one or more of the above criteria of remission are met is considered a significant patient response. Such responses, although not indicative of total remission, are indicators of a favorable response to treatment.

It will be understood that the treatment of the present invention may not achieve total remission in any or all patients treated in accordance therewith, the degree of remission depending on the nature and degree of the disease in any particular patient.

In an embodiment of the present invention, the treatment of the invention for the induction of remission in patients suffering from UC which have been unresponsive to 5-ASA results in, 5-ASA Cₘₐₓ value greater than about 2800 ng/ML and an area under the curve (AUC) greater than about 21,000 ng.h/mL for a period between about 6 to about 18 weeks or until the treated patient obtains a UC-DAI score ≤1.

Formulations useful in the treatment of the present invention exhibit a single dose *in vivo* plasma concentration profile substantially the same as that shown in Figure 1. The latter shows the Arithmetic Mean (± SD) (over about 100 patients) plasma concentration profile achieved for 5-ASA in MMX® mesalamine in normal, healthy subjects. The formulations of the instant invention should achieve substantially the same mean plasma concentration curves as those shown in Figure 1 and Table 2 below. By substantially the same "profile" herein is meant that two curves have substantially the same AUC (area under the curve) and Cₘₐₓ, *e.g*., these parameters for each curve are ±20% of each other, or even closer, *e.g* ±10%, ±5%, ±2%, etc., which parameters are conventionally determined. See, *e.g*., Fundamentals of Clinical Pharmacokinetics. J. G. Wagner, Drug Intelligence Publications, Inc., Hamilton, III., 1975; Guidance for Industry, Bioavailability and Bioequivalence Studies for Orally Administered Drug Products-General Considerations, FDA, CDER. October 2000.

### Examples

### Example 1

The induction of remission of mild-to-moderate UC during therapy with MMX^{®} mesalamine 2.4g/day once or twice daily (QD or BID) (4.8g/day QD) was evaluated in two phase III, placebo-controlled, randomized studies (SPD476-301 and -302). Study 302 also included an active internal reference arm (Asacol^{®} 2.4g/day given three times daily).

Remission was defined in the studies using stringent criteria as a modified UC-DAI score of ≤ calculated as scores of 0 for rectal bleeding and stool frequency, a combined Physician's Global Assessment and sigmoidoscopy score of ≤1, no mucosal friability and at least a 1-point reduction in sigmoidoscopy score from baseline. Patients who did not achieve remission in studies 301 or 302 could opt to receive an additional 8 weeks' therapy (at a dose of 4.8g/day given as 2.4g BID) as part of an open-label study (SPD476-303). This part of the study was labeled the Acute, or Extension, Phase.

The UC-DAI consisted of rectal bleeding, stool frequency and sigmoidoscopy scores and PGA. Each of these parameters was assessed on a scale of 0 to 3, with 3 being the most severe score. The sum of scores for all four parameters determined the UC-DAI score. Assessment of sigmoidoscopic appearance was performed in the worst inflamed area in the rectum or in the sigmoid if the rectum was not inflamed. The same area was evaluated throughout the study. The sigmoidoscopy and PGA were performed by the same investigator/endoscopist.

Patients were recruited into the Acute (Extension) Phase (Study 303) if they were not in remission (UC-DAI remission criteria not met) at the end of studies 301 or 302. Patients in the Acute (Extension) Phase received MIX® mesalamine 4.8g/day (2.4g dosed BID) for 8 weeks. These patients visited the clinic on three occasions over 2 months.

At week 8 of the 301 and 302 studies, significantly more patients achieved remission in the MMX® mesalamine 2.4 g/day group compared with the placebo group (37.2% vs. 17.5% [P<0.001]). Significantly more patients in the MMX® mesalamine 4.8g/day group also achieved remission compared with the placebo group (35.1% vs. 17.5% [P<0.001]). However, a number of patients failed to achieve remission. Employing the further treatment of the present invention over 50% of the patients that had not achieved remission after 8 weeks of treatment with either 2.4 g/day (1.2 g BID or 0.8 g TID) or 4.8 g/day (4.8 g QD) achieved remission by the further 8 week treatment with 4.8 g/day of 5-ASA (2.4 g BID)..

Specifically, as shown in Table 1 below, 61.9% of the patients from the 301 study that did not achieve remission after 8 weeks of 2.4 g 5-ASA therapy (administered as 1.2 g BID of MMX mesalamine) that then received an additional 8 weeks of 5-ASA therapy (4.8 g/day, administered as 2.4 g BID of MMX mesalamine) achieved remission.

Additionally, as further shown in Table 1, 69.6% of the patients from the 301 study who did not achieve remission after 8 weeks of 4.8 g mesalamine therapy (administered as 4.8 g QD of MMX mesalamine) but then received an additional 8 weeks of 5-ASA therapy (4.8 g/day, administered as 2.4 g BID of MMX mesalamine) achieved remission.

Similarly, as also shown in Table 1, 66.7% of the patients from the 302 study who did not achieve remission after 8 weeks of 5-ASA therapy (administered as 4.8 QD of MMX mesalamine), who then received an additional 8 weeks of 5-ASA therapy (4.8 g/day, administered as 2.4 g BID of MMX mesalamine) achieved remission.

Furthermore, as shown in Table 1, 71.4% of the patients from the 302 study who did not achieve remission after 8 weeks of 5-ASA therapy (administered as 2.4 g QD MMX mesalamine) and who then received an additional 8 weeks of 5-ASA therapy (4.8 g/day, administered as 2.4 g BID of MMX mesalamine) achieved remission.

As further shown in Table 1, 68.2% of the patients from the 302 study who did not achieve remission after 8 weeks of 5-ASA therapy (administered as 0.8 g TID Asacol®) but then received an additional 8 weeks of mesalamine therapy (4.8 g/day, administered as 2.4 g BID of MMX mesalamine) achieved remission.

**Table 1**

| **Percent of Patients in Remission following the Acute Phase treatment with MMX@ mesalamine** | | | | | | | |
|---|---|---|---|---|---|---|---|
| | Original Treatment (from Study 301) | | | Original Treatment (from Study 302) | | | |
| | | 2.4 g/d | 4.8 g/d | | 2.4 g/d | 4.8 g/d | 2.4 g/d |
| | | MMX | MMX | | MMX | MMX | Asacol |
| | | (1.2 BID) | (4.8 QD) | | (2.4 QD) | (4.8 QD) | (0.8 TID) |
| Study 303 4.8g/d (2.4g BID) | | 61.9% | 69.6% | | 71.4% | 66.7% | 68.2% |
| N= | | 21 | 23 | | 21 | 15 | 22 |

The results in Table 1 show the percentage of patients who, having failed to achieve remission in the initial, pivotal 301 and 302 trials (*i.e*., the trials that established safety and efficacy), achieved remission after completion of an additional 8 weeks of treatment (the Acute Phase of Study 303). During the Acute (Extension) Phase, patients were given therapy using a high dose of MMX® mesalamine) (4.8 g/day, administered as 2.4 g BID) for an additional period of 8 weeks, which is 8 weeks longer than the typical course of 5-ASA treatment. These high remission rates occurred in patients who initially failed and would normally have been given steroids or other escalation therapies, such as corticosteroids or other immunologic treatments. It was also discovered that the high doses of 5-ASA administered in the studies did not give rise to any substantial side effects as might be expected by increasing the dosage of the 5-ASA active agent.

The present data show that additional time on therapy with a higher dose of MMX® mesalamine (4.8g/d given 2.4g BID) can induce remission in a majority of patients, including those in whom the original 5-ASA treatment would typically have been considered failures. The present results demonstrate that more "aggressive" 5-ASA therapy can induce remission in patients who otherwise fail and require alternative therapies.

### Example 2

The administration of single and multiple doses of MMX® mesalamine to normal, healthy human patients gave rise to the plasma concentration-time profiles shown in Figure 1. The profiles observed for 5-ASA after doses of 2.4g/day or 4.8g/day of the product are similar in shape with only the expected difference in magnitude due to the dose difference (See Figures 1 and 2)..

**Table 2**

| **a) Mean ± SD 5-ASA Pharmacokinetic parameters for single doses MMX® mesalamine** | | | | | | |
|---|---|---|---|---|---|---|
| **Dosage** | **t_{lag}** | **tₘₐₓ** | **Cₘₐₓ** | **AUC₀₋ₜ** | **AUC_{0-∞}** | **t_{1/2}** |
| g QD | h | h | ng/mL | ng.h/mL | ng.h/mL | h |
| 2.4 | 4.00 | 8.04 | 2932 | 18573 | 19852 | 7.41 |
| | (1.99- 18.0) | (4.00 - 48.0) | ± | ± | ± | ± |
| | | | **2957** | 10969 | 11740 | 4.65 |
| 4.8 | 4.00 | | 4385 | 47785 | 48141 | 6.28 |
| | (2.00- 16.0) | 8,04 | ± | ± | ± | ± |
| | | (6.00 - 32.1) | 3033 | 22421 | 25627 | 5.31 |

| | | | | | | |
|---|---|---|---|---|---|---|
| Median (range) | | | | | | |

**b) Mean ± SD 5-ASA Pharmacokinetic parameters at steady state for multiple doses of MMX® mesalamine**

| **Dosage** | **t_{lag}** | **tₘₐₓ** | **Cₛₛₘₐₓ** | **Cₛₛₘᵢₙ** | **AUCₛₛ** |
|---|---|---|---|---|---|
| g QD | h | h | ng/mL | ng/mL | ng.h/mL |
| 2.4 | 0 | 8.00 | 2918 | 660 | 22319 |
| | (0-0) | (0-22.0) | ± | ± | ± |
| | | | 2164 | 528 | 13697 |
| 4.8 | 0 | 8.50 | 5280 | 1424 | 49559 |
| | (0-4.0) | (6.00-22.0) | ± | ± | ± |
| | | | 3146 | 1261 | 23780 |

| | | | | | |
|---|---|---|---|---|---|
| Median (range) | | | | | |

It will be understood that various changes may be made in the embodiments described above without departing from the present invention, the scope of which will be determined by the following claims.

The present application and invention further includes the subject matter of the following numbered clauses:
1. A method of treating a subject suffering from inflammatory bowel disease comprising administering an initial daily dose of about 2.4-4.8 g of 5-ASA in controlled release form, wherein when remission is not achieved after such treatment for about 4-8 weeks, changing the initial dose to a modified daily dosage equal to or greater than about 4g of 5-ASA in controlled release form for a further period sufficient to achieve a favorable response to treatment.
2. The method of clause 1, wherein the inflammatory bowel disease is ulcerative colitis.
3. The method of clause 2, wherein the ulcerative colitis is active mild-to-moderate ulcerative colitis.
4. The method of clause 1, wherein the controlled release form of 5-ASA is Multi Matrix System mesalamine.
5. The method of clause 1, wherein the modified daily dose is administered for about 8 weeks.
6. The method of clause 1, wherein the modified daily dose is continued until an ulcerative colitis-disease activity index (UC-DAI) of about 1 or less is obtained in the subject.
7. The method of clause 1, wherein the modified daily dose is continued until a sigmoidoscopy score of about 1 or less is obtained in the subject.
8. The method of clause 1, wherein the modified daily dose is continued until a Physician's Global Assessment (PGA) of about 1 or less or less is obtained in the subject.
9. The method of clause 1, wherein the modified daily dose is continued until a UC-DAI of about 1 or less is obtained, a rectal bleeding and stool frequency score of 0 is obtained and there is at least about a 1 point reduction in the sigmoidoscopy score in the subject.
10. The method of clause 9, wherein the modified daily dose is about 2.4 g of 5-ASA administered twice a day and is continued until a combined Physician's Global Assessment and sigmoidoscopy score about 1 or less is obtained and no mucosal friability is detected in the subject.
11. The method of clause 1, wherein the initial daily dose is about 2.4 g of 5-ASA in controlled release form, administered as about a 1.2 g twice-daily dose, about a 2.4 g once-daily dose or about a 0.8 g thrice-daily dose, or wherein the initial daily dose is about 4.8 g of 5-ASA in controlled release form, administered as about a 4.8 g once-daily dose.
12. The method of clause 11, wherein the modified dosage is a dose of about 4.8 g of 5-ASA in controlled release form administered for up to about 8 weeks.
13. A method of treating inflammatory bowel disease in a human suffering from inflammatory bowel disease and who has been unresponsive to 5-ASA treatment, comprising administering a daily oral dose of at least about 2 g of 5-ASA for a period of about 4-10 weeks in controlled release form so as to obtain a Cₘₐₓ greater than about 2800 ng/ML and an AUC greater than about 21000 ng.h/mL or until the patient treated is in remission.

## Claims

1. 5-ASA for use in the treatment of inflammatory bowel disease comprising administering an initial daily of (i) 2.4 g of 5-ASA in controlled release form, administered as: a 1.2 g twice-daily dosage; a 2.4 g once-daily dosage; or a 0.8 g thrice-daily dose, or (ii) 4.8 g of 5-ASA in controlled release form, administered as a 4.8 g once-daily dosage, and , wherein when remission is not achieved after such treatment for 4-8 weeks, changing the initial dose to a modified daily dosage of 4.8 g of 5-ASA in controlled release form administered as 2.4 g BID for up to 8 weeks..

2. 5-ASA for use in the treatment of inflammatory bowel disease according to claim 1, wherein the inflammatory bowel disease is ulcerative colitis.

3. 5-ASA for use in the treatment of inflammatory bowel disease according to claim 2, wherein the ulcerative colitis is active mild-to-moderate ulcerative colitis.

4. 5-ASA for use in the treatment of inflammatory bowel according to claim 1, wherein the controlled release form of 5-ASA is Multi Matrix System mesalamine.

5. 5-ASA for use in the treatment of inflammatory bowel disease according to claim 1, wherein the modified daily dose is administered for 8 weeks.

6. 5-ASA for use in the treatment of inflammatory bowel according to claim 1, wherein the modified daily dose is continued until an ulcerative colitis-disease activity index (UC-DAI) of 1 or less is obtained in the subject.

7. 5-ASA for use in the treatment of inflammatory bowel disease according to claim 1, wherein the modified daily dose is continued until a sigmoidoscopy of 1 or less is obtained in the subject.

8. 5-ASA for use in the treatment of inflammatory bowel disease according to claim 1, wherein the modified daily dose is continued until a Physician's Global Assessment (PGA) of 1 or less or less is obtained in the subject.

9. 5-ASA for use in the treatment of inflammatory bowel disease according to claim 1, wherein the modified daily dose is continued until a UC-DAI of 1 or less is obtained, a rectal bleeding and frequency score of 0 is obtained and there is at least a 1 point reduction in the sigmoidoscopy score in the subject.

10. 5-ASA for use in the treatment of inflammatory bowel disease according to claim 9, wherein the modified daily dose is 2.4 g of 5-ASA administered twice a day and is continued until a combined Physician's Global Assessment and sigmoidoscopy score 1 or less is obtained and no mucosal friability is detected in the subject.

11. 5-ASA for use in the treatment of inflammatory bowel disease in patients in whom remission is not achieved after treatment for 4-8 weeks with a total of 2.4 g or 4.8g of 5-ASA per day in controlled release form in the form of a once, twice or thrice daily dosage, wherein said treatment of patients includes changing the initial dose to a modified daily dosage of 4.8 g of 5-ASA in controlled release form administered as 2.4 g BID for up to 8 weeks.
